# EUROPÄISCHE PATENTANMELDUNG

(11) **EP 4 018 917 A1**
(43) Veröffentlichungstag der Anmeldung: **29.06.2022**
(21) Anmeldenummer: 21217915.4
(22) Anmeldetag: 28.12.2021
(51) Int. Cl.: A61B 5/00

(54) **SET AUS EINER KÖRPERHALTERUNG UND SENSOREINHEIT**

(30) Priorität: 28.12.2020 DE 102020134938
(71) Anmelder: Motesque, Inc., New Castle, Delaware 19808 (US)
(72) Erfinder: Fink, Tobias Cornelius, 52134 Herzogenrath (DE)
(74) Vertreter: Bauer, Dirk

(57) **Zusammenfassung**

Die Erfindung betrifft ein Set 1 aus einer Körperhalterung 2 und einer Sensoreinheit 3, bestehend aus einer Körperhalterung 2, die mit einem Körperteil eines Menschen in Kraft übertragender Weise verbindbar, insbesondere zur Umschlingung des Körperteils geeignet, ist und werkzeuglos davon lösbar ist, und einer Sensoreinheit 3, die ein Sensorgehäuse 4 und einen darin angeordneten Sensor und eine Übertragungseinrichtung aufweist, mit dem Sensordaten drahtlos übertragbar sind, wobei die Körperhalterung 2 aus einer Trägereinheit 6 mit einer daran angeordneten Aufnahme für das Sensorgehäuse 4 und einer an gegenüber liegenden Seiten der Trägereinheit 6 an diese angeschlossenen Umschlingungseinheit 7 besteht, mit der das Körperteil umschlingbar ist, die eine um das umschlungene Körperteil umlaufende Längsachse 8 besitzt und die zwei freie Enden aufweist, die in einem umschlungenen Zustand mit einander verbindbar sind.

Um ein Set aus einer Körperhalterung 2 und einer Sensoreinheit 3 bereitzustellen, mittels dem eine Relativbewegung zwischen einem umschlungenen Köperteil und dem daran befindenden Set aus einer Körperhalterung 2 und einer Sensoreinheit 3 verringert wird, wird erfindungsgemäß vorgeschlagen, dass die Umschlingungseinheit 7 mindestens ein Stabgelenkteil 10 aufweist, das eine Mehrzahl von quer zu der Längsachse 8 angeordneten, an ihren Enden gelenkig miteinander verbundenen Stäben, aufweist, wobei eine in Richtung der Längsachse 8 gemessene Länge des Stabgelenkteils 10 durch in Richtung der Längsachse 8 verlaufende Zugkräfte elastisch vergrößerbar ist. Außerdem ist das mindestens eine Stabgelenkteil 10 einstückig mit der Trägereinheit 6 verbunden und die Trägereinheit 6 und das mindestens eine Stabgelenkteil 10 sind als ein Kunststoff-Spritzgussteil ausgebildet.

## Beschreibung

Die Erfindung betrifft ein Set aus einer Körperhalterung und einer Sensoreinheit gemäß dem Oberbegriff von Anspruch 1.

### Stand der Technik

Im Stand der Technik sind Sets aus einer Körperhalterung mit einer Sensoreinheit in verschiedensten Ausführungsformen bekannt. Mit derartigen Sets werden insbesondere Beschleunigungsdaten während eines Bewegungsablaufs der sie tragenden Person, z. B. einer sich auf einem Laufband mit einem zu testenden Schuh bewegenden Person, erfasst und entweder gespeichert oder vorzugsweise zu einem Empfangsgerät mit Auswerte- und Anzeigeeinrichtung übertragen, vorzugsweise drahtlos mittels Bluetooth oder WLAN. Die Anbringung der Sets erfolgt dabei im Bereich eines Schuhs, Fußes Sprunggelenks und/oder Unterschenkels.

Typische Ausführungsformen der bekannten Sets umfassen eine Körperhalterung, bestehend aus einer Trägereinheit, auf der eine Aufnahme für eine Sensoreinheit angeordnet ist, sowie einer Umschlingungseinheit, die vorzugsweise aus einem Gummiband oder Gummigurt besteht. Die Trägereinheit wird mittels der Umschlingungseinheit kraftschlüssig an einem Körperteil angebracht, sodass die Verbindung werkzeuglos anbringbar und lösbar ist.

In einer weiteren Ausführung besteht die Umschlingungseinheit zusätzlich aus Brackets, die an gegenüberliegenden Seiten der Körperhalterung gelenkig mit dieser verbunden sind. Das Bracket als solches ist in einer unelastischen Form ausgestaltet, sodass die Beweglichkeit ausschließlich als Ganzes und relativ zur Körperhalterung über Gelenke erreicht wird. Durch die unelastische Ausgestaltung der Brackets ist die Anpassungsfähigkeit der Körperhalterung an die verschiedenen umschlungenen Körperformen, insbesondere bei kleinen Körperteilquerschnitten, nachteilig. Nachteilig ist die verminderte Haftung zwischen dem Körperteil und der daran befindenden Körperhalterung, wodurch unerwünschte Relativbewegungen zwischen der Körperhalterung inklusive der Sensoreinheit und des Körperteils auftreten. Diese Relativbewegungen resultieren in von der Sensoreinheit erfassten Beschleunigungen, die nicht die tatsächlichen Beschleunigungen der Körperteile wiedergeben, sondern die ein "Eigenleben" der Sets widerspiegeln und die Datenqualität vermindern und außerdem in einem geringeren Tragekomfort für den Menschen, dessen Körperteil umschlungen ist.

Außerdem ist aus der US 6,254,551 B1 eine Vorrichtung zur Erfassung und Übermittlung von Sensorsignalen betreffend Körper- bzw. Vitalfunktionen bekannt. In einer Ausführungsform ist der Sensor mit einem Band oder Gurt an dem Körper einer Person angebracht. Dieses Band besitzt einen elastischen Bereich oder Abschnitt, der über seine Längenänderung eine Messfunktion ausübt. Eine Verhinderung von Relativbewegungen zwischen Körper und Sensoren ist bei diesem Stand der Technik weder erforderlich noch überhaupt angesprochen.

Die US 3,830,414 A und die US 2015/0164419 A1 offenbaren jeweils eine uhrartige Vorrichtung, die mittels eines Armbandes an dem Arm einer Person angebracht wird. In erstgenanntem Dokument ist ein elastisch dehnbarer Abschnitt aus einer Mehrzahl metallischer Elemente gebildet, deren konkreter Aufbau nicht näher erläutert ist. Bei der US 2015/0164419 A1 ist ein dehnbarer Abschnitt des Armbandes an dessen beiden dem Uhrgehäuse zugewandten Enden jeweils in Form doppelt-mäanderförmig verlaufender Stege vorhanden. Eine Stabilisierungsfunktion für das Uhrgehäuse können die Ausgleichsabschnitte nicht erfüllen.

### Aufgabe

Der Erfindung liegt mithin die Aufgabe zugrunde, das das Set aus der Körperhalterung und der Sensoreinheit der eingangs beschriebenen Art derart weiterzuentwickeln, dass eine Relativbewegung zwischen einem umschlungenen Köperteil und dem daran befindenden Set aus der Körperhalterung und der Sensoreinheit verringert wird.

### Lösung

Ausgehend von einem Set aus einer Körperhalterung und einer Sensoreinheit der eingangs beschriebenen Art wird die zugrunde liegende Aufgabe erfindungsgemäß dadurch gelöst, dass eine Umschlingungseinheit mindestens ein Stabgelenkteil aufweist, das eine Mehrzahl von quer zu einer Längsachse angeordneten Stäben aufweist, die an ihren Enden gelenkig miteinander verbundenen sind, wobei das mindestens eine Stabgelenkteil 10 einstückig mit der Trägereinheit 6 verbunden ist und wobei die Trägereinheit 6 und das mindestens eine Stabgelenkteil 10 als ein Kunststoff-Spritzgussteil ausgebildet sind.

Eine in Richtung der Längsachse gemessene Länge des Stabgelenkteils ist durch in Richtung der Längsachse verlaufende Zugkräfte elastisch vergrößerbar. Vorteilhafte Ausführungen ergeben sich aus den Merkmalen der Unteransprüche.

Die erfindungsgemäße Lösung ermöglicht, dass sich die Körperhalterung durch das mindestens eine Stabgelenkteil besser an eine Form eines umschlungenen Körperteils anpasst. Hierdurch wird eine bessere Haftung zwischen dem umschlungenen Körperteil und der daran befindenden Körperhalterung erreicht. Infolgedessen wird die Relativbewegung zwischen beiden vermindert. Dies führt einerseits zu einem höheren Tragekomfort für einen Menschen, an dem die Körperhalterung angebracht ist, als auch zu einer höheren Qualität der erfassten Daten, insbesondere werden Beschleunigungsspitzen, die durch unerwünschte Relativbewegungen zwischen Sensor und Körper der Person entstehen und das Messergebnis verfälschen, verhindert. Außerdem gewährleistet die Einstückigkeit von Trägereinheit und dem mindestens einen Stabgelenkteil, dass kein Spiel und somit keine Relativbewegung zwischen dem Trägerteil und der Umschlingungseinheit auftritt. Außerdem bietet die Einstückigkeit große fertigungstechnische Vorteile aufgrund reduzierter Werkzeug-, Material- und Montagekosten. Im Gegensatz zu metallischen Stabgelenkteilen, die aus vielen Einzelteilen zusammengesetzt sind, sind die Herstellungskosten von als Kunststoff-Spritzgussteile ausgebildeten einstückigen Einheiten aus Trägereinheit und Stabgelenkteil (oder -teilen) deutlich geringer.

Die verbesserte Haftung wird durch eine bessere Anpassungsfähigkeit der Körperhalterung, insbesondere des mindestens eine Stabgelenkteils, an die Form des umschlungenen Körperteils erreicht. Gelenke, welche dem mindestens einen Stabgelenkteils zugeordnet sind und dazugehörigen Stäbe miteinander verbinden, ermöglichen die Vergrößerung der Länge des Stabgelenkteils in Richtung der Längsachse, indem diese Stäbe auf Biegung belastet sind. Vorteilhafterweise ermöglichen die Gelenke zusätzlich eine zumindest teilweise Rotation der Stäbe zueinander um durch das jeweilige Gelenk und senkrecht zu der Längsachse verlaufende Rotationsachsen. Hierdurch kann sich die Körperhalterung entlang einer Umschlingungsrichtung an unterschiedliche Körperformen anpassen. Auch wenn die Gelenke grundsätzlich als klassische Drehgelenke mit klar definierten Drehachsen ausgebildet sein können, ist es bevorzugt, dass die Gelenke durch einfache, auf Biegung oder Torsion belastete Materialbrücken, d. h. Verbindungsquerschnitte, zwischen benachbarten Stabenden gebildet sind. Bei einer einstückigen Ausbildung von Trägereinheit und Stabgelenkteil, wie sie erfindungsgemäß vorliegt, ist dies der Fall.

Vorzugsweise schließt das mindestens eine Stabgelenkteil an dem der Trägereinheit abgewandten Ende lösbar an das Bandteil an, weiter vorzugsweise verbindet ein einziges Bandteil mit seinen beiden gegenüberliegenden Enden, vorzugsweise lösbar, die freien Enden zweier Stabgelenkteile miteinander. Die Stabgelenkteile übernehmen in diesem Fall nicht die Schließfunktion der Umschlingungseinheit, sondern bilden lediglich eine Längenausgleichs- und Stabilisierungsfunktion.

Eine vorteilhafte Ausgestaltung besteht darin, dass an gegenüberliegenden Seiten der Trägereinheit jeweils ein Stabgelenkteil angeordnet ist. Das beidseitige Vorhandensein von Stabgelenkteilen führt zu einer um die Längsachse betrachteten gleichmäßigeren und somit besseren Haftung des Sets aus der Körperhalterung und der Sensoreinheit an dem umschlungenen Körperteil. Dadurch wird eine Rotation des Sets entlang einer in den Mittelpunkt des umschlungenen Körperteils zeigenden, senkrecht zur Längsachse verlaufenden, Achse verhindert.

Vorteilhafterweise ist auch das Sensorgehäuse als Kunststoff-Spritzgussteil ausgebildet. Hierdurch wird eine schnelle Fertigung unter der Einhaltung geringer Fertigungstoleranzen ermöglicht. Insbesondere weist Kunststoff vorteilhafte elastische Eigenschaften sowie eine geringe Masse auf.

Eine Weiterbildung der Erfindung besteht darin, dass die Umschlingungseinheit mindestens ein flexibles, vorzugsweise gummielastisches, Bandteil aufweist, wobei weiter vorzugsweise an mindestens einem freien Ende des Bandteils vorzugsweise an jeweils einem freien Ende jedes Bandteils ein Verschlusselement angeordnet ist. Hierdurch wird eine verbesserte Anpassung des Bandteils mit der Form des umschlungenen Körperteils sowie eine kraftschlüssige Verbindung der Körperhalterung an dem umschlungenen Körperteil erreicht. Zusätzlich bietet die Ausgestaltung mit Gummi vorteilhafte Haftungseigenschaften.

Es ist möglich, dass das mindestens eine Stabgelenkteil mäanderförmig ausgebildet ist. Diese Ausgestaltung ist vorteilhaft, da eine Mäanderform eine in sich biegbare Struktur aufweist, sodass sich die einzelnen Stäbe der Mäanderform an die unterschiedliche Form des umschlungenen Körperteils anpassen können. Diese vorteilhafte Anpassungsfähigkeit verbessert die Haftung des mindestens einen Stabgelenkteils an dem umschlungenen Körperteil, wodurch die Relativbewegung zueinander verringert wird.

Vorteilhafterweise kann das mindestens eine Stabgelenkteil aus einer Mehrzahl von Rautengliedern zusammengesetzt sein. Jeweils vier in Form einer Raute angeordnete Stäbe bilden eine Doppelmäanderform. Hierdurch wird eine vorteilhafte Anpassungsfähigkeit und somit verbesserte Haftung des mindestens einen Stabgelenkteils an dem umschlungenen Körperteil erreicht. Infolgedessen wird die Relativbewegung zueinander verringert und die Sensordatenqualität verbessert.

Eine Weiterbildung der Erfindung besteht in einem Kopplungsbereich des mindestens einen Stabgelenkteils, in dem ein Ende des jeweilig zugeordneten Bandteils an das Stabgelenkteil angeschlossen ist, ein Hakenelement oder ein Ösenelement angeordnet ist, das mit mindestens einem komplementären Ösenelement oder Hakenelement des Bandteils koppelbar ist. Neben der Kopplung über eine Ösen-Haken-Verbindung sind auch Verbindungen z.B. in Form eines Spanngurts möglich. Hierdurch wird ein werkzeugloses Anbringen und Lösen des Bandteils an dem Stabgelenkteil und an dem umschlungenen Körperteil ermöglicht. Außerdem ermöglicht das mindestens eine Ösenelement oder Hakenelement des Bandteils die Umschlingungseinrichtungen auf verschiedene Körperteilquerschnitte anzupassen.

Eine Weiterbildung der Erfindung besteht darin, dass die Längsachse des Umschlingungsteils durch den mindestens einen Kopplungsbereich des mindestens einen Stabgelenkteils verläuft. Hierdurch wird die Trägereinheit inklusive der Sensoreinheit mittig auf der Längsachse angeordnet, sodass eine vorteilhafte Gewichtsverteilung der Trägereinheit sowie Sensoreinheit oberhalb und unterhalb der Längsachse entsteht.

### Infolgedessen wird

Eine weitere vorteilhafte Ausgestaltung besteht darin, dass eine dem Körperteil zugewandte Unterseite der Trägereinheit und/oder des mindestens einen Stabgelenkteils zumindest teilweise mit einem thermoplastischen Elastomer versehen ist, wobei vorzugsweise die Trägereinheit und/oder das mindestens eine Stabgelenkteil im Wege eines Zweikomponenten-Spritzgießverfahrens hergestellt ist bzw. sind. Das Aufbringen eines thermoplastischen Elastomers auf der dem Körperteil zugewandten Unterseite führt zu einer deutlichen Verbesserung der Haftung dieser Teile mit einer in kontaktstehenden Haut des umschlungenen Körperteils. Hierdurch wird die Relativbewegung zwischen umschlungenen Körperteil und der Sensoreinheit deutlich reduziert. Ferner ist die Ausführung in Form eines Zweikomponenten-Spritzgießverfahrens vorteilhaft, weil das Spritzengießen des thermoplastischen Elastomers ("Weichkomponente") als Schritt 2 ohne zusätzliche Montage oder Nachbehandlung in das Herstellungsverfahren integriert werden kann und das Werkstück nach dem Einspritzen der Hartkomponente nicht aus dem Werkzeug entfernt werden muss..

Vorteilhafterweise sind Gelenke zwischen benachbarten Stäben auf Biegung belastet werden. Die Belastung auf Biegung ermöglicht eine verbesserte Anpassungsfähigkeit des Stabgelenkteils, da jeder einzelne Stab sich der Form des umschlungenen Körperteils anpassen kann.

### Ausführungsbeispiel

Die Erfindung ist nachstehend anhand eines Ausführungsbeispiels, das in den Figuren dargestellt ist, näher erläutert. Es zeigt:
- Figur 1.: Eine Ansicht des erfindungsgemäßes Sets 1 aus einer Körperhalterung 2 und einer Sensoreinheit 3,
- Figur 2.: Eine Ansicht auf die Unterseite des Sets 1,
- Figur 3.: Einen Schnitt durch das Set 1 entsprechend der Schnittebene A-A in Figur 2.

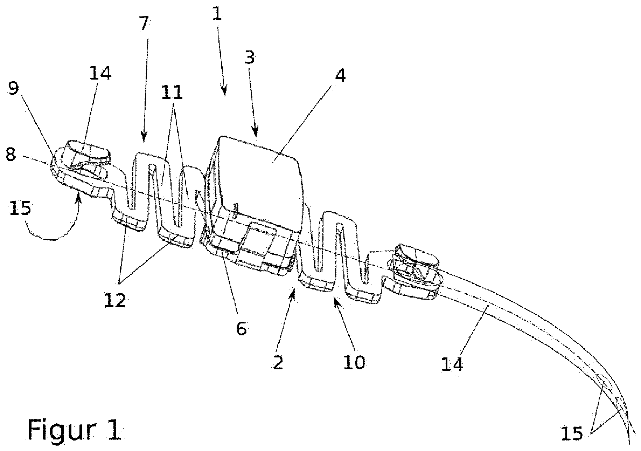

Ein Ausführungsbeispiel, das in Figur 1 bis Figur 3 gezeigt ist, umfasst ein erfindungsgemäßes Set 1 aus einer Körperhalterung 2 und einer Sensoreinheit 3, wobei die Körperhalterung 2 aus einer Trägereinheit 6 und einer Umschlingungseinheit 7 besteht. Die Umschlingungseinheit 7 wird aus einem Bandteil 13, welches vorzugsweise aus Gummi besteht, sowie zwei Stabgelenkteilen 10, die jeweils aus mehreren Stäben 11 mit dazwischen angebrachten Gelenken 12 bestehen, gebildet. Die Stabgelenkteile 10 sind an gegenüberliegenden Seiten an der Trägereinheit 6 angeordnet. Die Trägereinheit 6 sowie die Stabgelenkteile 10 sind einstückig mittels eines Kunststoff-Spritzguss-Verfahrens hergestellt und bilden ein einziges zusammenhängendes Spritzgussteil. Am Ende der Stabgelenkteile 10 befindet sind jeweils ein freies Ende 9, welches jeweils ein Hakenelement 14 aufweist, an denen das Bandteil 13 mit komplementären Ösenelementen 14 angebracht werden kann. Das Bandteil 13 weist hierzu an unterschiedlichen Positionen entlang der Längsachse 8 Ösenelemente 14 auf, wodurch die Umschlingungseinheit 7 auf einen Durchmesser eines umschlungenen Körperteils, z. B. eines Fußgelenks, angepasst werden kann.

Dazu wird das Set 1 an das zu umschlingendes Körperteil gelegt und das Bandteil 13, das bereits mit einem freien Ende 9 des Stabgelenkteils 10 verbunden ist, wird um das Körperteil geschlungen und an dem anderen freien Ende 9 des anderen Stabgelenkteils 10 so befestigt, dass sowohl das Bandteil 13, als auch die Stabgelenkteile 10 auf Zug belastet sind. Hierdurch wird die Umschlingungseinheit 7 entlang der Längsachse 8 um eine Strecke elastisch vergrößert, wobei die Gesamtverlängerung sowohl durch eine elstischer Dehnung der beiden Stabgelenkteile als auch eine elastische Dehnung des Bandteils 13 bewirkt wird. Dies ermöglicht das Umschlingen von Körperteilen mit unterschiedlichen Durchmessern, wobei gleichwohl stets eine angemessene Spannung zur sicheren Fixierung des Sets 1 an dem Körperteil realisierbar ist.

Somit können sich die einzelnen Stäbe 11 durch die Mäanderform der Stabgelenkteile 10 an unterschiedliche Ausprägungsformenformen des umschlungenen Körperteils entlang der Längsachse 8 des umschlungenen Körperteils anpassen. Hierdurch wird der Tragekomfort verbessert, insbesondere wird eine bessere Haftung zwischen Umschlingungseinheit 7 und dem Körperteil erreicht, wodurch eine Relativbewegung zwischen der Sensoreinheit 3 und umschlungenen Körperteil verringert wird. Die vergleichsweise große Breite der Stabgelenkteile 10 (gemessen quer zu der Längsachse 8 der Umschlingungseinheit 7) wird insbesondere auch eine relative Verdrehung zwischen der eine größere Dicke und Masse aufweisenden Trägereinheit 6 und dem jeweils einstückig angeschlossenen Stabgelenkteil 10 verhindert. Die jeweilige Verbindung zwischen Stabgelenkteil 10 und Trägereinheit 6 ist verdrehfest ausgebildet und besitzt einen entsprechend groß dimensionierten Verbindungsquerschnitt.

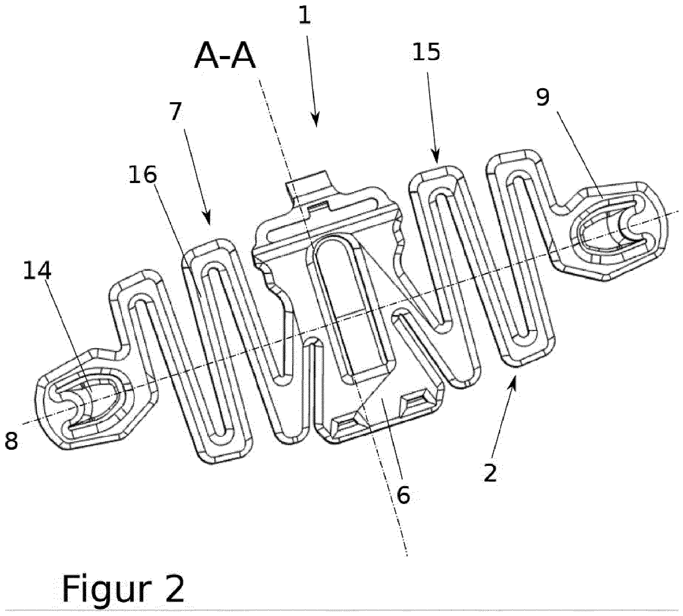

Zusätzlich kann eine vorzugsweise durchgehende thermoplastische Elastomerschicht 16 auf der Unterseite 15 der Trägereinheit 6 und/oder der Stabgelenkteile 10 aufgebracht werden. Hierdurch wird die Haftung zwischen dem Stabgelenkteil 10 und/oder der Trägereinheit 6 und der in Kontakt mit dem thermoplastischen Elastomers 16 stehenden Haut des umschlungenen Körperteils verbessert. Insbesondere wird somit eine (translatorische) Relativbewegung zwischen dem umschlungenen Körperteil und der Sensoreinheit 3 verringert bzw. unterbunden. Diese Ausgestaltung ist in Figur 2 zu erkennen.

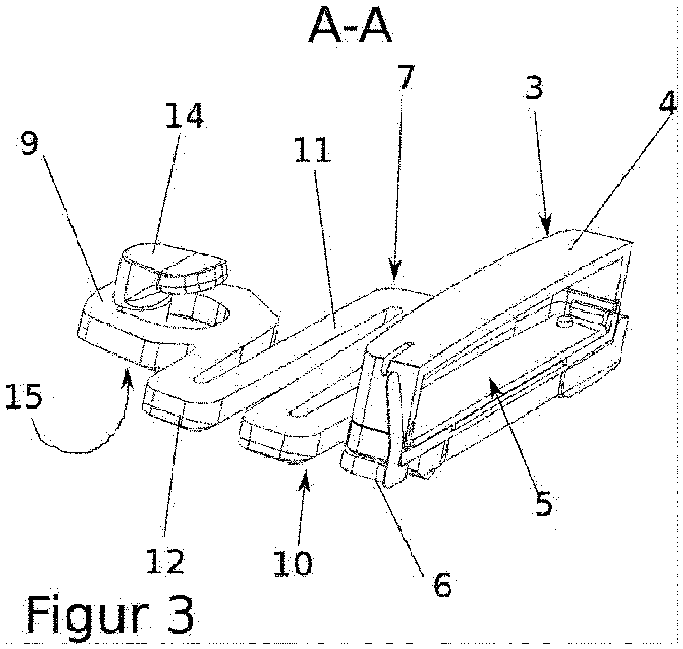

In Figur 3 ist ein Innenraum 5 des Sensorgehäuses 4 sichtbar, in dem sich ein Sensor sowie eine Übertragungseinrichtung, in einer entsprechend dem Stand der Technik bekannter Form, befindet. Sowohl der Sensor als auch die Übertragungseinrichtung sind nicht in den Figuren abgebildet. Gemäß der Erfindung handelt es sich um einen Sensor zum Messen von Beschleunigungen. Allerdings sind auch beliebige andere Sensortypen möglich. Das Sensorgehäuse 4) wird über eine Klick-Verbindung an der Trägereinheit 6 befestigt.

### Bezugszeichenliste

- 1.: Set
- 2.: Körperhalterung
- 3.: Sensoreinheit
- 4.: Sensorgehäuse
- 5.: Innenraum
- 6.: Trägereinheit
- 7.: Umschlingungseinheit
- 8.: Längsachse
- 9.: Freies Ende
- 10.: Stabgelenkteil
- 11.: Stab
- 12.: Gelenk
- 13.: Bandteil
- 14.: Haken- oder Ösenelement
- 15.: Unterseite
- 16.: Thermoplastisches Elastomer

## Patentansprüche

1. Set (1) aus einer Körperhalterung (2) und einer Sensoreinheit (3), bestehend aus
- einer Körperhalterung (2), die mit einem Körperteil eines Menschen in Kraft übertragender Weise verbindbar, insbesondere zur Umschlingung des Körperteils geeignet, ist und werkzeuglos davon lösbar ist, und
- einer Sensoreinheit (3), die ein Sensorgehäuse (4) und einen darin angeordneten Sensor und eine Übertragungseinrichtung aufweist, mit dem Sensordaten drahtlos übertragbar sind,
wobei die Körperhalterung (2) aus
- einer Trägereinheit (6) mit einer daran angeordneten Aufnahme für das Sensorgehäuse (4) und
- einer an gegenüber liegenden Seiten der Trägereinheit (6) an diese angeschlossenen Umschlingungseinheit (7) besteht,
○ mit der das Körperteil umschlingbar ist,
○ die eine um das umschlungene Körperteil umlaufende Längsachse (8) besitzt und
○ die zwei freie Enden (9) aufweist, die in einem umschlungenen Zustand mit einander verbindbar sind,
wobei die Umschlingungseinheit (7) mindestens ein Stabgelenkteil (10) aufweist, das eine Mehrzahl von quer zu der Längsachse (8) angeordneten, an ihren Enden gelenkig miteinander verbundenen Stäben (11), aufweist, wobei eine in Richtung der Längsachse (8) gemessene Länge des Stabgelenkteils (10) durch in Richtung der Längsachse (8) verlaufende Zugkräfte elastisch vergrößerbar ist **dadurch gekennzeichnet, dass** das mindestens eine Stabgelenkteil 10 einstückig mit der Trägereinheit 6 verbunden ist und dass die Trägereinheit 6 und das mindestens eine Stabgelenkteil 10 als ein Kunststoff-Spritzgussteil ausgebildet sind.

2. Set (1) nach Anspruch 1, **dadurch gekennzeichnet, dass** an gegenüber liegenden Seiten der Trägereinheit (6) jeweils ein Stabgelenkteil (10) angeordnet ist.

3. Set (1) nach einem der vorhergehenden Ansprüche, **dadurch gekennzeichnet, dass** das Sensorgehäuse (4) als Kunststoff-Spritzgussteil ausgebildet ist.

4. Set (1) nach einem der vorhergehenden Ansprüche, **dadurch gekennzeichnet, dass** die Umschlingungseinheit (7) mindestens ein flexibles, vorzugsweise gummielastisches, Bandteil (13) aufweist, wobei weiter vorzugsweise an mindestens einem freien Ende (9) eines Bandteils (13) vorzugsweise an jeweils einem freien Ende (9) jedes Bandteils (13) ein Verschlusselement (14) angeordnet ist.

5. Set (1) nach einem der vorhergehenden Ansprüche, **dadurch gekennzeichnet, dass** das mindestens eine Stabgelenkteil (10) eine Mäanderform aufweist.

6. Set (1) nach einem der Ansprüche 1 bis 5, **dadurch gekennzeichnet, dass** das mindestens eine Stabgelenkteil (10) aus einer Mehrzahl von Rautengliedern zusammengesetzt ist, die jeweils aus vier in Form einer Raute angeordneten Stäben bestehen.

7. Set (1) nach einem der vorhergehenden Ansprüche, **dadurch gekennzeichnet, dass** in einem Kopplungsbereich des mindestens einen Stabgelenkteils (10), in dem ein Ende des jeweils zugeordneten Bandteils (13) an das Stabgelenkteil (10) angeschlossen ist, ein Hakenelement oder ein Ösenelement (14) angeordnet ist, das mit mindestens einem komplementären Ösenelement oder Hakenelement (14) des Bandteils (13) koppelbar ist.

8. Set (1) nach Anspruch 8, **dadurch gekennzeichnet, dass** die Längsachse (8) des Umschlingungsteils (7) durch den mindestens einen Kopplungsbereich des mindestens einen Stabgelenkteils (10) verläuft.

9. Set (1) nach einem der vorhergehenden Ansprüche, **dadurch gekennzeichnet, dass** eine dem Körperteil zugewandte Unterseite (15) der Trägereinheit 6 und/oder des mindestens einen Stabgelenkteils (10) zumindest teilweise mit einem thermoplastischen Elastomer (16) versehen ist, wobei vorzugsweise die Trägereinheit (6) und/oder das mindestens eine Stabgelenkteil (10) im Wege eines Zweikomponenten-Spritzgießverfahrens hergestellt ist bzw. sind.

10. Set (1) nach einem der vorherigen Ansprüche, **dadurch gekennzeichnet, dass** Gelenke (12) zwischen benachbarten Stäben (10) auf Biegung belastet werden.
